# EUROPEAN PATENT APPLICATION

(11) **EP 4 458 983 A1**
(43) Date of publication of application: **06.11.2024**
(21) Application number: 22914857.2
(22) Date of filing: 28.12.2022
(51) Int. Cl.: C12Q 1/68, C07K 14/35, C12N 15/31, C07K 14/315

(54) **SINGLE MOLECULE NANOPORE SEQUENCING METHOD**

(30) Priority: 30.12.2021 CN 202111657436
(71) Applicant: BGI Shenzhen, Shenzhen, Guangdong 518083 (CN)
(72) Inventor: XU, Xun, Shenzhen, Guangdong 518083 (CN); DONG, Yuliang, Shenzhen, Guangdong 518083 (CN); JI, Zhouxiang, Shenzhen, Guangdong 518083 (CN); WANG, Lele, Shenzhen, Guangdong 518083 (CN); ZENG, Tao, Shenzhen, Guangdong 518083 (CN); LI, Yuxiang, Shenzhen, Guangdong 518083 (CN); CHEN, Ao, Shenzhen, Guangdong 518083 (CN); ZHANG, Wenwei, Shenzhen, Guangdong 518083 (CN)
(74) Representative: Burger, Hannes Alfred
(86) International application number: PCT/CN2022/142615
(87) International publication number: WO 2023/125605

(57) **Abstract**

A method for sequencing polynucleotides by using nanopores. The present invention relates to, in particular, a method for sequencing polynucleotides by means of covalently binding a polynucleotide binding protein and a nanopore.

## Description

### Technical Field

The present application relates to a method for sequencing polynucleotide using nanopore. Specifically, the present application relates to a method for sequencing polynucleotide by means of covalently binding a polynucleotide-binding protein to a nanopore.

### Background Art

Next generation sequencing technology (NGS) has laid the foundation for the development of genomics and accelerated the research and development of fundamental scientific research and clinical medicine. However, NGS still has many problems, such as expensive instruments, large memory and high cost for data processing, and short read length.

The third-generation single-molecule nanopore sequencing technology has been developed. Nanopore sequencing has the characteristics of high speed, ultra-long read length, and no need for amplification and replication by a polymerase. The sequencing speed can reach hundreds of bases per second, and the length of single sequencing read can be tens of kilobases, and the assembly of more continuous and complete genome sequence can be provided. The principle of nanopore sequencing is that: a transmembrane protein with a diameter of nanometer scale is inserted into a biomimetic membrane to form a nanometer-sized protein channel (nanopore) on the membrane; two electrodes are placed on both sides of the membrane; after electricity is applied, ions pass through the transmembrane protein to generate an ion current. The substance to be tested passes through the transmembrane protein under the action of an electric field, which causes characteristic changes in the ion current, and alters the magnitude of the current (current blockage). Different substances to be tested produce different current blockages.

In typical nanopore sequencing, when a sequencing library is captured by a nanopore, a motor protein slides through a spacer under the action of an electric field, and sequencing begins. The sequencing library, constructed with an adapter-motor protein complex where the spacer comprised in the adapter blocks the movement of the motor protein, cannot be preserved for a long time after library construction due to the limited stability and short storage period of the motor protein. Moreover, the spacer is composed of multiple modified nucleotides, which are more expensive than conventional nucleotides.

Therefore, there is a need for an improved single-molecule nanopore sequencing method with reduced cost, increased accuracy, and/or higher efficiency.

### Contents of the present application

The third-generation single-molecule nanopore sequencing technology comprises library construction and sequencing. Currently, a typical method comprises linking an adapter-motor protein complex to a target nucleic acid fragment to be tested to construct a sequencing library. Wherein, the adapter is in the shape of "Y" and is composed of top strand and bottom strand. The top strand contains the following three parts: (a) a leader sequence, which can facilitate the faster movement of the sequencing library towards the nanopore under an electric field force; (b) a spacer, which can prevent the motor protein from moving along the single-stranded polynucleotide and unwinding double-stranded polynucleotide; (c) a region complementary to a partial sequence of the bottom strand, a single-stranded polynucleotide containing a thymidine deoxynucleotide (dT) at the 3' end. The bottom strand comprises two parts: (i) a region complementary to the top strand, and (ii) a region complementary to a tether sequence. The tether comprises a cholesterol molecule at one end for anchoring a biomimetic membrane. During subsequent sequencing, the tether is incubated with the biomimetic membrane for anchorage. The sequencing library can be bound to the tether. After applying a certain voltage, the leader sequence is captured by the transmembrane protein; the motor protein moves through the spacer under an electric field force. During the sequencing process, the double-stranded polynucleotide is unwound into single-stranded polynucleotide by the motor protein, and the single-stranded polynucleotide passes through and interacts with the transmembrane protein embedded in the biomimetic membrane under an electric field force, which causes characteristic changes in ion current.

DNA has a fast speed when passing through the transmembrane protein, and the electrical signals generated have poor resolution. The motor protein can significantly reduce the speed at which DNA passes through the transmembrane protein, and the motor protein only hydrolyze one nucleotide at a time. The function of the spacer is to block the motor protein from moving along the single-stranded polynucleotide and unwinding the double-stranded polynucleotide in the sequencing buffer; the blockage is disabled under an electric field force when the sequencing library is captured by the transmembrane protein.

However, the sequencing library, constructed with an adapter-motor protein complex where the spacer comprised in the adapter blocks the movement of the motor protein, cannot be preserved for a long time after library construction due to the limited stability and short storage period of the motor protein. Moreover, the spacer is composed of multiple modified nucleotides, which are more expensive than conventional nucleotides.

The present application provides an improved single-molecule nanopore sequencing method, thereby providing the following aspects.

### Construct

Accordingly, in one aspect, the present application provides a construct comprising a transmembrane protein pore subunit and a polynucleotide-binding protein, wherein the subunit retains its pore-forming ability, the polynucleotide-binding protein is capable of unwinding the two strands of a double-stranded polynucleotide and/or controlling the movement of a single-stranded polynucleotide through the pore; and, the subunit is in covalent binding to the polynucleotide-binding protein.

In certain embodiments, the covalent bonding is not via a peptide bond generated by nucleic acid translation.

In certain embodiments, the covalent binding is via an isopeptide bond.

In certain embodiments, the covalent binding occurs through chemical ligation (e.g., by click chemistry).

In certain embodiments, the subunit binds to the polynucleotide-binding protein through an isopeptide bond.

In particular, the inventors of the present application discovered that the transmembrane protein and the polynucleotide-binding protein provided by the present application are ligated through an isopeptide bond, which has greater advantages than other ligation methods.

For example, when chemical ligation is used to form a transmembrane protein/polynucleotide-binding protein complex, and then the complex is inserted into the membrane to form a single-channel nanopore, the inventors discovered that the transmembrane protein or polynucleotide-binding protein should be modified to adapt to this chemical ligation method, which increases complexity and difficulty; at the same time, after the chemical ligation reaction is completed, further purification is required to obtain the transmembrane protein/polynucleotide-binding protein complex to remove the reagents required for the chemical reaction. Reagents for the chemical reaction may be unfriendly to the activity of the transmembrane protein or the polynucleotide-binding protein. At the same time, without purification, these reagents will often affect subsequent experiments. For example: some compounds may pass through the transmembrane protein under the electric field force, which may interfere with the current signals as detected; or, some compounds may affect the stability of phospholipid membrane in subsequent experiments, which may cause single-channel nanopore failure.

On the other hand, when the modified porin is inserted into the phospholipid bilayer membrane to form a single-channel nanopore, a polynucleotide-binding protein is then added, and after incubation, a transmembrane protein/polynucleotide-binding protein complex is formed through chemical ligation, the inventors found that the ligation between the transmembrane protein and the polynucleotide-binding protein needs to be carried out in the nanopore detection reaction chamber, and an appropriate voltage needs to be applied on both sides of the membrane to detect whether a transmembrane protein/polynucleotide-binding protein complex is formed. If the chemical ligation reaction is performed in the chemical reaction chamber for sequencing, additives or necessary components added to achieve the chemical ligation may affect the stability of the phospholipid membrane or the detection of whether the transmembrane protein/polynucleotide-binding protein complex is formed, thereby affecting subsequent further detection.

In the present application, the ligation of isopeptide bond does not require the addition of additional substances, and there is no interference factor for sequencing reagents or detection reagents in subsequent further experiments.

In certain embodiments, the isopeptide bond is formed from a protein-protein binding pair, and the protein-protein binding pair consists of a first member (e.g., a first peptide tag) and a second member (e.g., a second peptide tag), wherein the first member and the second member are linked via an isopeptide bond, and, the first member is linked to the N-terminal or C-terminal of the transmembrane protein pore subunit optionally via a first linker (e.g., a rigid or flexible linker, such as a peptide linker comprising one or more glycines and/or one or more serines) to form a first component, and the second member is linked to the N-terminal or C-terminal of the polynucleotide-binding protein optionally via a second linker (e.g., a rigid or flexible linker, such as a peptide linker comprising one or more glycines and/or one or more serines) to form a second component.

In certain embodiments, the first linker is a peptide linker having the amino acid sequence as set forth in SEQ ID NO: 15.

In certain embodiments, the second linker is a peptide linker having the amino acid sequence as set forth in SEQ ID NO: 14.

In certain embodiments, the protein-protein binding pair is selected from the group consisting of: SpyCatcher/SpyTag pair, SpyTag002/SpyCatcher002 pair, SpyTag003/SpyCatcher003 pair, isopeptag-N/pilin-N pair, isopeptag/pilin-C pair, and SnoopTag/Snoop-Catcher pair.

In certain embodiments, the isopeptide bond is formed by a SpyTag003/SpyCatcher003 pair.

In certain embodiments, the SpyCatcher003 is linked to the N-terminal of the subunit.

In certain embodiments, the SpyTag003 is linked to the N-terminal of the polynucleotide-binding protein.

In certain embodiments, the SpyCatcher003 has the amino acid sequence as set forth in SEQ ID NO: 4.

In certain embodiments, the SpyTag003 has the amino acid sequence as set forth in SEQ ID NO: 1.

In certain embodiments, the subunit is selected from subunits derived from hemolysin, MspA, Frac, ClyA, PA63, CsgG, GspD, XcpQ, Wza, SP1, Phi29 connector, SPP1 connector, T3 connector, T4 connector, T7 connector, potassium ion channel protein, sodium ion channel protein, and calcium ion channel protein.

In certain embodiments, the subunit is further linked with an additional polypeptide, and the additional polypeptide is selected from the group consisting of tag, enzyme cleavage site, signal peptide, guide peptide, detectable label, and any combination thereof.

In certain embodiments, the subunit has the amino acid sequence as set forth in SEQ ID NO: 3 or 17. The sequence shown here does not contain an amino acid (e.g., methionine (Met)) encoded by a start codon (e.g., ATG) at its N-terminal. Those skilled in the art understand that in the process of preparing proteins through genetic engineering, due to the role of the start codon, the first position of the polypeptide chain produced is often the amino acid encoded by the start codon (e.g., Met). The subunit of the present application not only includes subunits having an amino acid sequence that does not contain the amino acid encoded by the start codon (e.g., Met) at its N-terminal, but also includes subunits having an amino acid sequence that contains the amino acid encoded by the start codon (e.g., Met) at its N-terminal. Therefore, the sequences that further contain an amino acid (e.g., Met) encoded by a start codon at the N-terminal of the above amino acid sequence are also within the scope of the present application.

In certain embodiments, the polynucleotide-binding protein is selected from the group consisting of nucleic acid helicases, such as DNA helicases or RNA helicases.

In certain embodiments, the polynucleotide-binding protein is selected from Dda, UvrD, Rep, RecQ, PcrA, eIF4A, NS3, Rep, gp41 and T7gp4.

In certain embodiments, the polynucleotide-binding protein is further linked with an additional polypeptide, and the additional polypeptide is selected from the group consisting of tag, enzyme cleavage site, signal peptide, guide peptide, detectable label, and any combination thereof.

In certain embodiments, the polynucleotide-binding protein has the amino acid sequence as set forth in SEQ ID NO: 2 or 16.

### Pore for sequencing polynucleotide

In another aspect, the present application also provides a pore for sequencing polynucleotides, which comprises at least one construct as described above.

In certain embodiments, the pore is a transmembrane protein pore.

In certain embodiments, the pore comprises at least one construct as described above and other subunit required to form the pore.

In certain embodiments, the pore comprises a sufficient number of the other subunits required to form the pore.

In certain embodiments, the other subunit required to form the pore is the same or different from the transmembrane protein pore subunit in the construct.

In certain embodiments, the other subunit required to form the pore is the same as the transmembrane protein pore subunit in the construct, or the other subunit required to form the pore is a paralog, homolog or variant of the transmembrane protein pore subunit in the construct.

In certain embodiments, the pore comprises one construct as described above and other subunit required to form the pore.

In certain embodiments, the pore comprises a sufficient number of the other subunits required to form the pore.

In certain embodiments, the pore is formed from one construct and seven the other subunits required to form the pore.

In certain embodiments, the other subunit required to form the pore is the same as or different from the transmembrane protein pore subunit in the construct.

In certain embodiments, the other subunit required to form the pore is the same as the transmembrane protein pore subunit in the construct, or, the other subunit required to form the pore is a paralog, homolog or variant of the transmembrane protein pore subunit in the construct.

### Isolated nucleic acid

In another aspect, the present application also provides an isolated nucleic acid, which encodes the construct as described above.

In certain embodiments, the isolated nucleic acid molecule comprises a first nucleotide sequence encoding a first component and a second nucleotide sequence encoding a second component, the first component and the second component are as defined above, the first nucleotide sequence and the second nucleotide sequence are present on the same or different isolated nucleic acid molecules.

In certain embodiments, the first component is a first protein formed by linking the first member to the N-terminal or C-terminal of the transmembrane protein pore subunit optionally via a first peptide linker; and the second component is a second protein formed by linking the second member to the N-terminal or C-terminal of the polynucleotide-binding protein optionally via a second peptide linker.

### Vector

In another aspect, the present application also provides a vector, which comprises the isolated nucleic acid molecule as described above. In certain embodiments, the vector is a cloning vector or an expression vector.

### Host cell

In another aspect, the present application also provides a host cell, which comprises the isolated nucleic acid molecule as described above or the vector as described above.

### Preparation method

In certain embodiments, the first component is a first protein formed by linking the first member to the N-terminal or C-terminal of the transmembrane protein pore subunit optionally via a first peptide linker; and the second component is a second protein formed by linking the second member to the N-terminal or C-terminal of the polynucleotide-binding protein optionally via a second peptide linker.

In such embodiment, the present application also provides a method for preparing the construct or the pore as described above.

The method for preparing the construct as described above comprises:
culturing a host cell containing a first nucleotide sequence encoding a first component and a second nucleotide sequence encoding a second component under a condition that allows protein expression, the first component and the second component being as defined above, and, recovering the construct from a culture of the cultured host cell; wherein the first component comprising a transmembrane protein pore subunit and the second component comprising a polynucleotide-binding protein are capable of forming a complex linked by an isopeptide bond during the expressing and/or recovering process;
   or,
culturing a first host cell containing a first nucleotide sequence encoding a first component, and a second host cell containing a second nucleotide sequence encoding a second component under a condition that allows protein expression, respectively, to obtain the first component comprising a transmembrane protein pore subunit and the second component comprising a polynucleotide-binding protein, respectively, the first component and the second component being as defined above; incubating the first component and the second component to form a complex linked by an isopeptide bond.

The method for preparing the pore as described above comprises the following steps:
(1) contacting a first component with other subunit required to form the pore to form a multimer, the multimer being a pore that does not contain a polynucleotide-binding protein, wherein the first component is as defined above, the other subunit required to form the pore is as defined above;
(2) contacting the multimer with a second component, the second component being as defined above, so that the second component is linked to the first component in the multimer via an isopeptide, to form a pore linked with a polynucleotide-binding protein.

In certain embodiments, the multimer described in step (1) is an octamer formed from one first component and seven other subunits required to form the pore.

In certain embodiments, the subunit in the first component is the same as the other subunit required to form the pore.

In certain embodiments, step (1) comprises the following steps: culturing a host cell containing a first nucleotide sequence encoding the first component and a third nucleotide sequence encoding the other subunit required to form the pore, to obtain a pore that does not contain the polynucleotide-binding protein.

In certain embodiments, step (1) further comprises a step of purifying the pore that does not contain the polynucleotide-binding protein.

In certain embodiments, the first component comprises a first purification tag and the other subunit required to form the pore comprises a second purification tag.

In certain embodiments, the first purification tag is different from the second purification tag, for example, the first purification tag is a His tag and the second purification tag is a Strep tag.

In certain embodiments, in step (1) of the method for preparing the pore, the pore that does not contain the polynucleotide-binding protein is purified by the first purification tag and the second purification tag. In certain embodiments, in step (1) of the method for preparing the pore, the pore that does not contain the polynucleotide-binding protein and is formed with one first component and seven other subunits required to form the pore is purified by the first purification tag and the second purification tag.

In some embodiments, after step (1), the method for preparing the pore further comprises a step of removing the first purification tag and/or the second purification tag.

### Sequencing method

In another aspect, the present application also provides a method for sequencing a target polynucleotide, which comprises:
(a) providing the pore as described above, and a polynucleotide to be tested;
(b) contacting the polynucleotide to be tested with the pore, so that the polynucleotide-binding protein of the pore binds to the polynucleotide to be tested, and the polynucleotide-binding protein controls a single-stranded polynucleotide to which it binds to pass through the pore; and,
(c) obtaining one or more measurement values as the single-stranded polynucleotide moves relative to the pore, wherein the one or more measurement values are capable of being used to character sequence information of the polynucleotide to be tested.

In certain embodiments, in step (a), the polynucleotide to be tested is a double-stranded polynucleotide, and the polynucleotide-binding protein unwinds the double-stranded polynucleotide to provide a single-stranded polynucleotide, and the polynucleotide-binding protein controls the single-stranded polynucleotide to pass through the pore; the double-stranded polynucleotide comprises at least one single-stranded overhang (e.g., a 5'-end overhang and/or a 3'-end overhang), and the single-stranded overhang comprises a leader sequence, and the leader sequence guides the single-stranded polynucleotide to which it links to enter the pore.

In certain embodiments, the double-stranded polynucleotide may contain a gap and/or a non-terminal overhang.

In certain embodiments, the leader sequence generally enters the pore along the field generated by the applied electric potential.

In certain embodiments, the leader sequence generally comprises a polymer. The polymer is preferably negatively charged. The polymer is preferably a polynucleotide, such as DNA or RNA, modified nucleotide (e.g., abasic DNA), PNA, LNA, polyethylene glycol (PEG), or polypeptide.

In certain preferred embodiments, the leader sequence comprises a single-stranded polynucleotide. In certain embodiments, the leader sequence comprises a single-stranded DNA sequence, such as a poly dT. In certain preferred embodiments, the leader sequence is 10 to 150 nucleotides in length, such as 20 to 150 nucleotides in length. One skilled in the art can adjust the length of the leader sequence according to the pore used.

In certain embodiments, the polynucleotide-binding protein controls the single-stranded polynucleotide to move through the pore in the direction from 5' end to 3' end, and, the double-stranded polynucleotide comprises at least one 5'-end overhang, and the 5'-end overhang comprises the leader sequence.

It is easy for those skilled in the art to understand that "single-stranded polynucleotide moves through the pore in the direction from 5' end to 3' end" refers to that the nucleotide residues comprised from 5' end to 3' end in the single-stranded polynucleotide pass through the pore sequentially.

In certain embodiments, the polynucleotide-binding protein controls the single-stranded polynucleotide to move through the pore in the direction from 3' end to 5' end, and the double-stranded polynucleotide comprises at least one 3'-end overhang, and the 3'-end overhang comprises the leader sequence.

It is easy for those skilled in the art to understand that "single-stranded polynucleotide moves through the pore in the direction from 3' end to 5' end" refers to that the nucleotide residues comprised from 3' end to 5' end in the single-stranded polynucleotide pass through the pore sequentially.

In certain embodiments, the polynucleotide to be tested is a linear double-stranded polynucleotide.

In certain embodiments, the linear double-stranded polynucleotide comprises at least one single-stranded overhang (e.g., a 5'-end overhang and/or a 3'-end overhang) at each of its two ends, and the single-stranded overhang comprises the leader sequence.

In certain embodiments, the linear double-stranded polynucleotide comprises at least one single-stranded overhang (e.g., a 5'-end overhang and/or a 3'-end overhang) at its one end, and the single-stranded overhang comprises the leader sequence; and, the linear double-stranded polynucleotide comprises a bridge portion at its other end, and the bridge portion covalently links the two single strands of the linear double-stranded polynucleotide. In certain embodiments, the bridging portion covalently links the ends of the two single strands of the linear double-stranded polynucleotide. For example, the bridging portion covalently links the 3' end (or 5' end) of one single strand of the linear double-stranded polynucleotide to the 5' end (or 3' end) of the other single strand of the linear double-stranded polynucleotide.

In certain embodiments, the bridging portion is a hairpin-shaped oligonucleotide adapter. In certain embodiments, the linear double-stranded polynucleotide containing the oligonucleotide adapter behaves essentially as a hairpin-containing duplex formed from one oligonucleotide strand.

In certain embodiments, the polynucleotide to be tested is a circular double-stranded polynucleotide.

In certain embodiments, the circular double-stranded polynucleotide is obtained by rolling circle amplification using a primer containing the leader sequence and a carrier sequence as a template.

In some embodiments, the amplification process and the sequencing process are performed in the same system. For example, the circular double-stranded polynucleotide is unwound into two single strands by the polynucleotide-binding protein, one of the two single strands passes through the pore, and the other strand serves as a template to generate a new circular double-stranded polynucleotide.

In certain embodiments, in step (a), the polynucleotide to be tested is a single-stranded polynucleotide, and the polynucleotide-binding protein controls the single-stranded polynucleotide to move through the pore; wherein, the single-stranded polynucleotide comprises a leader sequence at least at one of its two ends, and the leader sequence guides the single-stranded polynucleotide to which it links to enter the pore. In certain embodiments, the polynucleotide-binding protein controls the single-stranded polynucleotide to move through the pore in the direction from 5' end to 3' end, and the single-stranded polynucleotide comprises the leader sequence at least at its 5' end.

It is easy for those skilled in the art to understand that "single-stranded polynucleotide moves through the pore in the direction from 5' end to 3' end" refers to that the nucleotide residues comprised from 5' end to 3' end in the single-stranded polynucleotide pass through the pore sequentially.

In certain embodiments, the polynucleotide-binding protein controls the single-stranded polynucleotide to move through the pore in the direction from 3' end to 5' end, and the single-stranded polynucleotide comprises the leader sequence at least at its 3' end.

It is easy for those skilled in the art to understand that "single-stranded polynucleotide moves through the pore in the direction from 3' end to 5' end" refers to that the nucleotide residues comprised from 3' end to 5' end in the single-stranded polynucleotide pass through the pore sequentially.

In certain embodiments, the polynucleotide to be tested is linked to the outer edge or vicinity of the pore.

In certain embodiments, one or more tethers are conjugated to the outer edge or vicinity of the pore, the tether comprises a capture sequence having sequence complementarity to a portion of the polynucleotide to be tested, and the hybridization of the capture sequence with the complementary portion in the polynucleotide to be tested allows the polynucleotide to be tested to be linked to the outer edge or vicinity of the pore.

In certain embodiments, when the polynucleotide to be tested is a double-stranded polynucleotide as defined above, the portion of the polynucleotide to be tested complementary to the capture sequence comprises a portion of the single-stranded overhang or a portion of the strand that is aligned with the single-stranded overhang. In certain embodiments, the portion of the polynucleotide to be tested complementary to the capture sequence comprises a portion of the single-stranded overhang. In certain embodiments, the portion of the single-stranded overhang is located 5' or 3' of the leader sequence. In certain preferred embodiments, the portion of the single-stranded overhang passes through the pore after the leader sequence. In certain embodiments, the portion of the polynucleotide to be tested complementary to the capture sequence comprises a portion of a strand that is aligned with the single-stranded overhang.

As used herein, the term "strand that is aligned with the single-stranded overhang" refers to a strand that is hybridized with (i) an oligonucleotide strand at which the single-stranded overhang is located, or, (ii) a strand that is hybridized to the same strand with which the oligonucleotide stand located with the single-stranded overhang hybridizes.

In certain embodiments, when the polynucleotide to be tested is a single-stranded polynucleotide as defined above, the portion of the polynucleotide to be tested complementary to the capture sequence comprises a portion of the single-stranded polynucleotide.

In some embodiments, one or more tethers are conjugated to the outer edge or vicinity of the pore, the tether comprises the capture sequence, and the capture sequence is linked to the polynucleotide to be tested via a linker; wherein, the linker comprises a first region complementary to the capture sequence, and a second region having sequence complementarity to a portion of the polynucleotide to be tested.

In certain embodiments, when the polynucleotide to be tested is a double-stranded polynucleotide as defined above, the portion of the polynucleotide to be tested complementary to the capture sequence comprises a portion of the single-stranded overhang or a portion of a strand that is aligned with the single-stranded overhang. In certain embodiments, the portion of the polynucleotide to be tested complementary to the capture sequence comprises a portion of the single-stranded overhang. In certain embodiments, the portion of the single-stranded overhang is located 5' or 3' of the leader sequence. In certain preferred embodiments, the portion of the single-stranded overhang passes through the pore after the leader sequence. In certain embodiments, the portion of the polynucleotide to be tested complementary to the capture sequence comprises a portion of a strand that is aligned with the single-stranded overhang.

In certain embodiments, when the polynucleotide to be tested is the single-stranded polynucleotide as defined above, the portion of the polynucleotide to be tested complementary to the capture sequence comprises a portion of the single-stranded polynucleotide.

In certain embodiments, the pore is disposed in a membrane.

In certain embodiments, the membrane is an amphiphilic layer, such as a lipid bilayer (e.g., phospholipid bilayer) or a high molecular polymer membrane (e.g., di-block polymer membrane, tri-block polymer membrane).

In certain embodiments, the tether is covalently or non-covalently linked to the pore or a portion of the membrane near to the pore.

In certain embodiments, the tether is anchored to a portion of the membrane near to the pore via a cholesterol molecule.

In certain embodiments, the cholesterol molecule is linked to the capture sequence of the tether via a spacer molecule. In certain embodiments, the spacer molecule consists of 4×iSp18.

It is readily understood that the tether is used to specifically or non-specifically enrich the polynucleotide to be tested in the vicinity of the pore. Therefore, those skilled in the art can design the length, number and capture sequence (specific or non-specific capture sequence) of the tether as needed, and the present application does not limit this.

In certain embodiments, step (b) and step (c) are performed in a solution.

In certain embodiments, the solution is ionic and the measurement value is an ionic current through the nanopore.

In certain embodiments, in step (b), a potential difference is provided across the nanopore to allow the single-stranded nucleic acid to enter the nanopore.

### Device

In another aspect, the present application also provides a device for sequencing a target polynucleotide, comprising: (i) a membrane; (ii) a plurality of transmembrane protein pores in the membrane, wherein the transmembrane protein pore is selected from the group consisting of the pores as described above.

In certain embodiments, the device further comprises instructions for performing the method for sequencing the target polynucleotide as described above.

In certain embodiments, the membrane is an amphiphilic layer, such as a lipid bilayer (e.g., phospholipid bilayer) or a high molecular polymer membrane (e.g., di-block polymer membrane, tri-block polymer membrane).

In certain embodiments, the device does not comprise a polynucleotide-binding protein provided separately from (ii).

### Kit

In another aspect, the present application also provides a kit, which comprises the construct, pore, isolated nucleic acid molecule, vector, and/or host cell as described above.

In certain embodiments, the kit further comprises a reagent for nanopore sequencing.

### Use

In another aspect, the present application also provides a use of the construct, pore, isolated nucleic acid molecule, vector, host cell, device, or kit as described above for sequencing a polynucleotide.

### Definition of Terms

In the present application, unless otherwise stated, scientific and technical terms used herein have the meanings commonly understood by those skilled in the art. Moreover, the procedures of virology, biochemistry, and immunology laboratory used herein are routine procedures widely used in the corresponding fields. Meanwhile, in order to better understand the present application, definitions and explanations of relevant terms are provided below.

When the terms "for example," "e.g.," "such as," "comprising," "including," or variations thereof are used herein, these terms will not be considered limiting terms and will instead be interpreted to mean "not limited to" or "without limitation."

Unless otherwise indicated herein or clearly contradicted by context, the terms "a" and "an" as well as "the" and similar referents in the context of describing the present application (especially in the context of the following claims) are to be construed to cover singular and plural.

As used herein, the terms "to anneal," "annealing," "anneal," "hybridize," or "hybridizing" and the like refer to the formation of complex between nucleotides having sufficient complementarity to form a complex via Watson-Crick base pairing. For the purposes of the present application, nucleic acid sequences that are "complementary" or "complementary to" or "hybridize" or "anneal" to each other should be capable of forming a sufficiently stable "hybrid" or "complex" to serve the intended purpose. It is not required that every nucleic acid base within the sequence displayed by a first nucleic acid molecule is capable of base pairing or pairing or complexing with every nucleic acid base within the sequence displayed by a second nucleic acid molecule such that the two nucleic acid molecules or corresponding sequences as shown therein are "complementary" or "anneal" or "hybridize" to each other. As used herein, the term "complementary" or "complementarity" is used when referring to sequences of nucleotides that are connected by base pairing rules. For example, the sequence 5'-A-G-T-3' is complementary to the sequence 3'-T-C-A-5'. Complementarity can be "partial"', wherein only some of the nucleic acid bases match according to the rules of base pairing. Optionally, there can be 'complete' or 'full' complementarity between nucleic acids.

As used herein, the term "polynucleotide" such as nucleic acid is a macromolecule containing two or more nucleotides. A polynucleotide or nucleic acid may comprise any combination of any nucleotides. The nucleotides may be naturally occurring or synthetic. The nucleotides may be modified or not. In certain embodiments, the nucleotides contain no modifications.

### Beneficial effects of the present application

The nanopore sequencing method of the present application has one or more of the following beneficial effects:
(1) The sequencing library constructed by the nanopore sequencing method of the present application does not contain motor proteins, and thus has a longer storage period, and can greatly reduce the requirements for library construction conditions (e.g., there is no need to consider whether the motor protein is denatured when constructing the library). In addition, since the sequencing library does not contain motor proteins, the sequencing method of the present application does not require the use of spacers, which can reduce costs. At the same time, in the present application, sequencing is started immediately after the sequencing library is captured by the transmembrane protein, which can improve detection efficiency.
(2) In particular, on the one hand, in the existing technology, the library construction method is subject to many limitations due to the presence of spacers (which are usually modified special nucleotides) in the library. For example, sequencing libraries cannot be constructed solely by conventional nucleic acid amplification. On the contrary, the nanopore sequencing method provided by the present application does not require the use of the spacer. Therefore, when constructing a sequencing library, more library construction methods can be used or they can be better combined with other technical solutions, such as rolling circle amplification. On the other hand, due to the greater inclusiveness of the library structure and construction mode of the method of the present application as mentioned above, in some embodiments, the sequencing process and the library construction process can be carried out in the same system, and can further reduce costs and improve efficiency.
(3) In addition, there may be multiple sites where the motor protein is bound to the nucleic acid to be tested, that is, an adapter may be bound with different numbers of motor proteins, resulting in differences in sequencing speed and other performance during sequencing. Therefore, if the library molecules contain motor proteins and spacers, the adapter-motor protein complex generally needs to be purified to obtain a unitary adapter-motor protein complex. The sequencing library provided by the present application that does not contain motor proteins and spacers can well avoid this problem.

The embodiments of the present application will be described in detail below with reference to the accompanying drawings and examples, but those skilled in the art will understand that the following drawings and examples are only used to illustrate the present application and do not limit the scope of the present application. Various objects and advantageous aspects of the present application will become apparent to those skilled in the art from the following detailed description of the accompanying drawings and preferred embodiments.

### Brief Description of the Drawings

Fig. 1 shows the schematic diagram of core construction of the expression vectors pET.28a(+)-6*His-Spytag003-DDA, pET.28a(+)-6*His-Spycatcher003-MspA and pET.21a(+)-strep-MspA.
Fig. 2 shows the schematic structural diagram of the complex of the motor protein and porin prepared in Example 1.
Fig. 3 shows the SDS-PAGE gel images of the complexes of Spycatcher003-MspA and MspA in different ratios purified by ion exchange in Example 1; in order not to destroy the polymer state of MspA, when preparing the sample, 5× non-denaturing and non-reducing protein loading buffer (P0016N, Beyotime) was added.
Fig. 4 shows the elution profile of the recombinant of the motor protein Spytag003-DDA and the porin Spycatcher003-MspA(MspA)₇ in Example 1 by size exclusion chromatography.

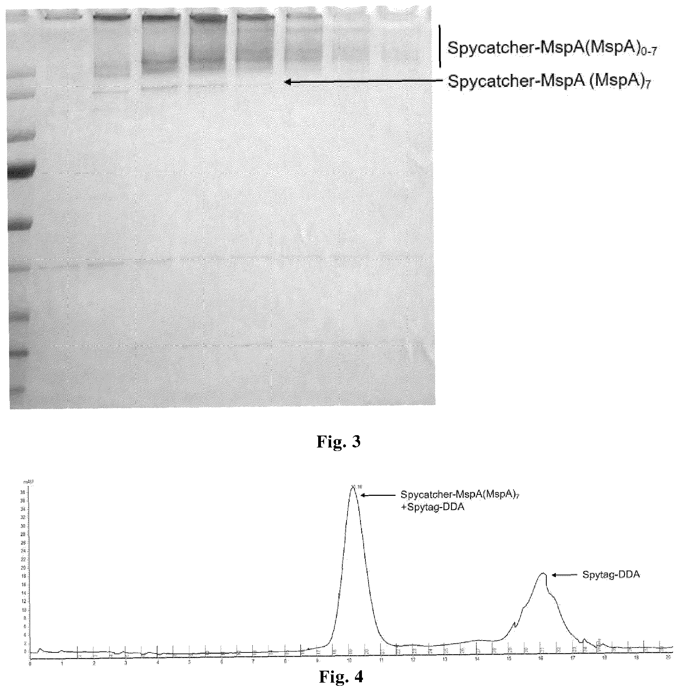

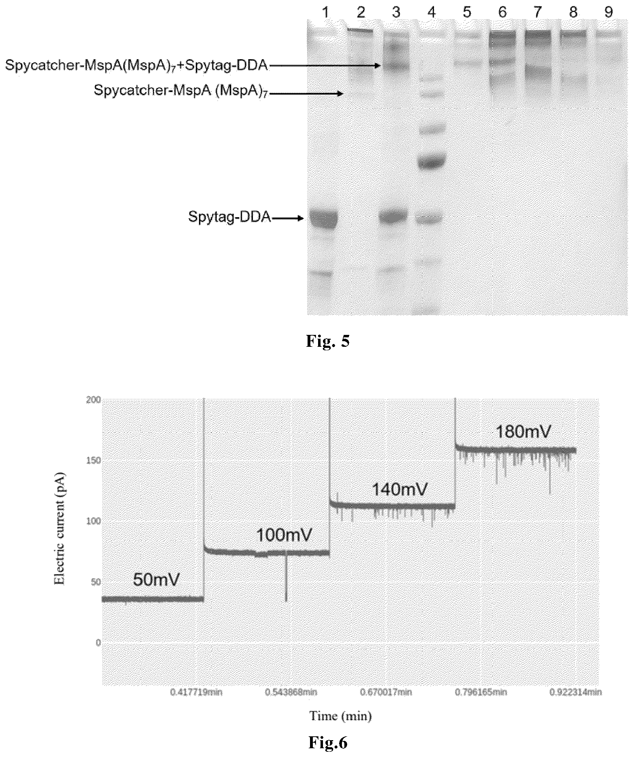

Fig. 5 shows the SDS-PAGE gel image of the recombinant of the motor protein Spytag003-DDA and porin Spycatcher003-MspA(MspA)₇ in Example 1; Lane 1 corresponds to the motor protein Spytag003-DDA alone. Lane 2 corresponds to the porin Spycatcher003-MspA(MspA)₇ alone. Lane 3 corresponds to the mixed sample after incubation of Spycatcher003-MspA(MspA)₇ and Spytag003-DDA before undergoing size exclusion chromatography. Lane 4 corresponds to the protein ladder (26616, Thermo Scientific). Lanes 5 to 9 correspond to the protein samples at elution peak of the complex of Spycatcher003-MspA(MspA)₇ and Spytag003-DDA after size exclusion chromatography, which correspond to the first peak in Fig. 4, of which lane 5 shows a single complex band.
Fig. 6 shows the open pore current distribution under different voltages (0.05V-0.1V-0.14V-0.18V) when the complex was inserted into membrane in Example 2.
Fig. 7 shows a schematic structural diagram of the double-stranded polynucleotide sequencing library constructed in Example 2 bound to the tether; wherein, 1 represents the leader sequence, including the motor protein-binding region, 2 represents the double-stranded DNA portion (including DNA fragment to be tested), and 3 represents the tether.
Fig. 8 shows the electrical signals when the double-stranded polynucleotide sequencing library constructed in Example 2 was unwound into single strands by Spytag003-DDA and moved and passed through the nanopore Spycatcher003-MspA(MspA)₇. Panel A in Fig. 8 shows a current amplitude diagram of sequencing events, and panel B in Fig. 8 shows a zoomed signal diagram of a section of panel A in Fig. 8.

### Sequence information

A description of the sequences involved in the present application is provided in the table below.

**Table 1: Sequence information**

| SEQ ID NO: | Description of sequence |
|---|---|
| 1 | Amino acid sequence of Spytag003 |
| | RGVPHIVMVDAYKRYK |
| 2 | Wild-type amino acid sequence of DDA helicase |
| | MTFDDLTEGQKNAFNIVMKAIKEKKHHVTINGPAGTGKTTLTKFIIEA- LISTGETGIILAAP- THAAKKILSKLSGKEASTIHSILKINPVTYECNVLFEQKEVPDLAKARV LICDEVSMYDRKLFKILLSTIPPWATIIGIGDNKQIRPVDPGENTAYISPF- FTHKDFYQCELTEVKRSNAPIIDVATDVRNGKWIYDKVVDGHGVRGF TGDTALRDFMVNYFSIVKSLDDLFENRVMAFTNKSVDKLNSIIRK- KIFETDKDFIVGEIIV- MQEPLFKTYKIDGKPVSEIIFNNGQLVRIIEAEYTSTFVKARGVPGEYLI RHWDLTVETYGDDEYYREKIKIISSDEELYKFNLFLGKT- CETYKNWNKGG- KAPWSDFWDAKSQFSKVKALPASTFHKAQGMSVDRAFIYTPCIHYAD VELAQQLLYVGVTRGRYDVFYV |
| 3 | Wild-type amino acid sequence of MspA |
| | GLDNELSLVDGQDRTLTVQQWDTFLNGVFPLDRNRLTREWFHS- GRAKYIVAGPGADE- FEGTLELGYQIGFPWSLGVGINFSYTTPNILINNGNITAPPFGLNSVITPN LFPGVSISARLGNGPGIQEVATFSVRVSGAKGGVAVSNAHGTVT- GAAGGVLLRPFARLIASTGDSVTTYGEPWNMN |
| 4 | Amino acid sequence of Spycatcher003 |
| | MVTTLSGLSGEQGPSGDMTTEEDSATHIKFSKRDEDGRELAGATMEL- RDSSGKTIST- WISDGHVKDFYLYPGKYTFVETAAPDGYEVATPIEFTVNEDGQVTVD GEATEGDAHT |
| 5 | Thrombin recognition sequence |
| | LVPRGS |
| 6 | Amino acid sequence of Strep tag |
| | WSHPQFEK |
| 7 | Amino acid sequence of Spytag003-DDA |
| | RGVPHIVMVDAYKRYKGGSGGSGGSGGSGGSGGSMTFDDLTEGQKN- AFNIVMKAIKEK- KHHVTINGPAGTGKTTLTKFIIEALISTGGTGIILAAPTHAAKKILSKLSG KEASTIHSILKINPVTYEENVLFEQKEVPDLAKCRVLICDE- VSMYDRKLFKILLSTIPPWC- TIIGIGDNKQIRPVEPGENTAYISPFFTHKDFYQCELTEVKRSNAPIIDVA TDVRNGKWNYDKVVDGHGVRGFTGDTALRDFMVNYFSIVKSLDD- LFENRVMAFTNKS- VDKLNSIIRKKIFETDKDFIVGEIIVMQEPLFKTYKIDGKPVSEIIFNNGQ LVRIIEAEYTSTFVKARGVPGEYLIRHWDLTVETYGDDEYYRE- KIKIISSDEELYKFNLFLAKTAETYKNWNKGGKAPWSDFWDAKSQFSK VKALPASTFHKAQGMSVDRAFIYTPCIHYADVELAQQL- LYVGVTRGRYDVFYV |
| 8 | Amino acid sequence of Spycatcher003-MspA |
| | MVTTLSGLSGEQGPSGDMTTEEDSATHIKFSKRDEDGRELAGATMEL- RDSSGKTIST- WISDGHVKDFYLYPGKYTFVETAAPDGYEVATPIEFTVNEDGQVTVD GEATEGDAHTGGSGGSGGSGLDNELSLVDGQDRTLTVQQWDTFLNG- VFPLDRNRLTREWFHS- GRAKYIVAGPGADEFEGTLELGYQIGFPWSLGVGINFSYTTPNILIDDG DITAPPFGLNSVITPNLFPGVSISADLGNGPGIQEVATFSVDVS- GAEGGVAVSNAHGTVT- GAAGGVLLRPFARLIASTGDSVTTYGEPWNMN |
| 9 | Amino acid sequence of Strep-MspA |
| | WSHPQFEKGALVPRGSGLDNELSLVDGQDRTLTVQQWDTFLNGVF- PLDRNRLTREWFHS- GRAKYIVAGPGADEFEGTLELGYQIGFPWSLGVGINFSYTTPNILIDDG DITAPPFGLNSVITPNLFPGVSISADLGNGPGIQEVATFSVDVS- GAEGGVAVSNAHGTVT- GAAGGVLLRPFARLIASTGDSVTTYGEPWNMN |
| 10 | Top strand of adapter |
| | TTTTTTTTTTTTTTTTTTTTTTGGTTGTTT- CTGTTGGTGCTGATATTGCT |
| 11 | Bottom strand of adapter |
| | GCAATATCAGCACCAACAGAAACAACCTTTGAGGCGAGCGGTCAA |
| 12 | Sequence of tether |
| | Cholesterol/iSp18/iSp18/iSp18/iSp18/TTGACCGCTCGCCTC |
| 13 | DNA sequence to be tested |
| | GGCGTAATCATGGTCATAGCTGTTTCCTGTGTGAAATTGT- TATCCGCTCACAATT- CCACACAACATACGAGCCGGAAGCATAAAGTGTAAAGCCTGGGGT GCCTAATGAGTGAGCTAACTCACATTAATTGCGTTGCGCTCA- CTGCCCGCTTTCCAGTCGG- GAAACCTGTCGTGCCAGCTGCATTAATGAATCGGCCAACGCGCGGG GAGAGGCGGTTTGCGTATTGGGCGCTCTTCCGCTTCCTCGCTCACT- GACTCGCTGCGCTCGGTCGTTCGGCTGCGGCGAGCGGTATCAGCTC ACTCAAAGGCGGTAATACGGTTATCCACAGAATCAGGG- GATAACGCAGGAAAGAACATGT- GAGCAAAAGGCCAGCAAAAGGCCAGGAACCGTAAAAAGGCCGCGT TGCTGGCGTTTTTCCATAGGCTCCGCCCCCCTGACGAGCATCA- CAAAAATCGACGCTCAAGT- CAGAGGTGGCGAAACCCGACAGGACTATAAAGATACCAGGCGTTT CCCCCTGGAAGCTCCCTCGTGCGCTCTCCTGTTCCGAC- CCTGCCGCTTACCGGATAC- CTGTCCGCCTTTCTCCCTTCGGGAAGCGTGGCGCTTTCTCATAGCTC ACGCTGTAGGTATCTCAGTTCGGTGTAGGTCGTT- CGCTCCAAGCTGGGCTGTGTGCACGAAC- CCCCCGTTCAGCCCGACCGCTGCGCCTTATCCGGTAACTATCGTCTT GAGTCCAACCCGGTAAGACACGACT- TATCGCCACTGGCAGCAGCCACTGGTAACAGGAT- TAGCAGAGCGAGGTATGTAGGCGGTGCTACAGAGTTCTTGAAGTGG TGGCCTAACTACGGCTACACTAGAAGAACAGTATTTGG- TATCTGCGCTCTGCTGAAGCCAGT- TACCTTCGGAAAAAGAGTTGGTAGCTCTTGATCCGGCAAACAAACC ACCGCTGGTAGCGGTGGTTTTTTTGTTTGCAAGCAGCAGATTA- CGCGCAGAAAAAAAG- GATCTCAAGAAGATCCTTTGATCTTTTCTACGGGGTCTGACGCTCAG TGGAACGAAAACTCACGTTAAGGGATTTTGGTCATGAGATTATCA- AAAAGGATCTTCACCTA- GATCCTTTTAAATTAAAAATGAAGTTTTAAATCAATCTAAAGTATAT ATGAGTAAACTTGGTCTGACAGTTACCAATGCTTAATCAGT- GAGGCACCTATCTCA- GCGATCTGTCTATTTCGTTCATCCATAGTTGCCTGACTCCCCGTCGT GTAGATAACTACGATACGGGAGGGCTTACCA- TCTGGCCCCAGTGCTGCAATGATAC- CGCGAGACCCACGCTCACCGGCTCCAGATTTATCAGCAATAAACCA GCCAGCCGGAAGGGCCGAGCGCAGAAGTGGTCCTGCAACTT- TATCCGCCTCCATCCAGTCTATTAATTGTTGCCGGGAAGCTAGAGTA AGTAGTTCGCCAGTTAATAGTTTGCGCAACGTTGTTGCCATT- GCTACAGGCATCGTGGTGT- CACGCTCGTCGTTTGGTATGGCTTCATTCAGCTCCGGTTCCCAACGA TCAAGGCGAGTTACATGATCCCCCATGTTGTGCAAAAAAGCGGT- TAGCTCCTT- CGGTCCTCCGATCGTTGTCAGAAGTAAGTTGGCCGCAGTGTTATCA CTCATGGTTATGGCAGCACTGCATAATTCTCTTACTGTCAT- GCCATCCGTAAGATGCTTTT- CTGTGACTGGTGAGTACTCAACCAAGTCATTCTGAGAATAGTGTAT |
| | GCGGCGACCGAGTTGCTCTTGCCCGGCGTCAATACGGGATAATAC- CGCGCCACATAGCAGAACTTTAAAAGTGCTCATCATTGGAAAACGT TCTTCGGGGCGAAAACTCTCAAGGATCTTACCGCTGTTGA- GATCCAGTTCGATGTAAC- CCACTCGTGCACCCAACTGATCTTCAGCATCTTTTACTTTCACCAGC GTTTCTGGGTGAGCAAAAACAGGAAGGCAAAAT- GCCGCAAAAAAGGGAATAAGGGCGACACG- GAAATGTTGAATACTCATACTCTTCCTTTTTCAATATTATTGAAGCA TTTATCAGGGTTATTGTCTCATGAGCGGATACATATTTGAATGTATT- TAG- AAAAATAAACAAATAGGGGTTCCGCGCACATTTCCCCGAAAAGTGC CACCTGACGTCTAAGAAACCATTATTATCATGACATTAAC- CTATAAAAATAGGCGTATCA- CGAGGCCCTTTCGTCTCGCGCGTTTCGGTGATGACGGTGAAAACCT CTGACACATGCAGCTCCCGGAGACGGTCACAGCTTGTCTGTAAGCG- GATGCCGG- GAGCAGACAAGCCCGTCAGGGCGCGTCAGCGGGTGTTGGCGGGTG TCGGGGCTGGCTTAACTATGCGGCATCAGAGCAGATTGTACT- GAGAGTGCACCATAT- GCGGTGTGAAATACCGCACAGATGCGTAAGGAGAAAATACCGCAT CAGGCGCCATTCGCCATTCAGGCTGCGCAACTGTTGG- GAAGGGCGATCGGTGCGGGCCTCTT- CGCTATTACGCCAGCTGGCGAAAGGGGGATGTGCTGCAAGGCGATT AAGTTGGGTAACGCCAGGGTTTTCCCAGTCACGACGTTG- TAAAACGACGGCCAGT |
| 14 | Amino acid sequence of Linker 1 |
| | GGSGGSGGSGGSGGSGGS |
| 15 | Amino acid sequence of Linker 2 |
| | GGSGGSGGS |
| 16 | Amino acid sequence of DDA helicase mutant |
| | MTFDDLTEGQKNAFNIVMKAIKEKKHHVTINGPAGTGKTTLTKFIIEA- LISTGGTGIILAAP- THAAKKILSKLSGKEASTIHSILKINPVTYEENVLFEQKEVPDLAKCRVL ICDEVSMYDRKLFKILLSTIPPWCTIIGIGDNKQIRPVEPGENTAYISPF- FTHKDFYQCELTEVKRSNAPIIDVATDVRNGKWNYDKVVDGHGVRGF TGDTALRDFMVNYFSIVKSLDDLFENRVMAFTNKSVDKLNSIIRK- KIFETDKDFIVGEIIV- MQEPLFKTYKIDGKPVSEIIFNNGQLVRIIEAEYTSTFVKARGVPGEYLI RHWDLTVETYGDDEYYREKIKIISSDEELYKFNLFLAKTAE- TYKNWNKGG- KAPWSDFWDAKSQFSKVKALPASTFHKAQGMSVDRAFIYTPCIHYAD VELAQQLLYVGVTRGRYDVFYV |
| 17 | Amino acid sequence of MspA mutant |
| | GLDNELSLVDGQDRTLTVQQWDTFLNGVFPLDRNRLTREWFHS- GRAKYIVAGPGADE- FEGTLELGYQIGFPWSLGVGINFSYTTPNILIDDGDITAPPFGLNSVITPN LFPGVSISADLGNGPGIQEVATFSVDVSGAEGGVAVSNAHGTVT- GAAGGVLLRPFARLIASTGDSVTTYGEPWNMN |

Note: In the tether sequence as set forth in SEQ ID NO: 12, cholesterol is linked to the 5' end of the polynucleotide through 4×iSp18, wherein "iSp 18" is an 18-atom hexa-ethyleneglycol spacer, and its structure is shown in Formula I:

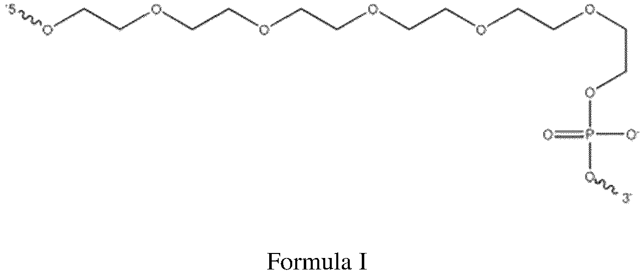

### Specific Models for Carrying Out the present application

The present application will now be described with reference to the following examples which are intended to illustrate but not to limit the present application.

Unless otherwise specified, the molecular biology experimental methods and immunoassay methods used in the present application were carried out basically according to the methods described by J. Sambrook et al., Molecular Cloning: Laboratory Manual, 2nd Edition, Cold Spring Harbor Laboratory Press, 1989, and F. M. Ausubel et al., Compiled Laboratory Guide to Molecular Biology, 3rd Edition, John Wiley & Sons, Inc., 1995; and the use of restriction enzymes was in accordance with the conditions recommended by the product manufacturer. Those skilled in the art will appreciate that the examples describe the present application by way of example and are not intended to limit the scope of the present application.

### Example 1: Expression and purification of Spytag003-containing motor protein DDA and MspA octamer labeled by Spycatcher003

### 1. Construction of recombinant expression plasmid

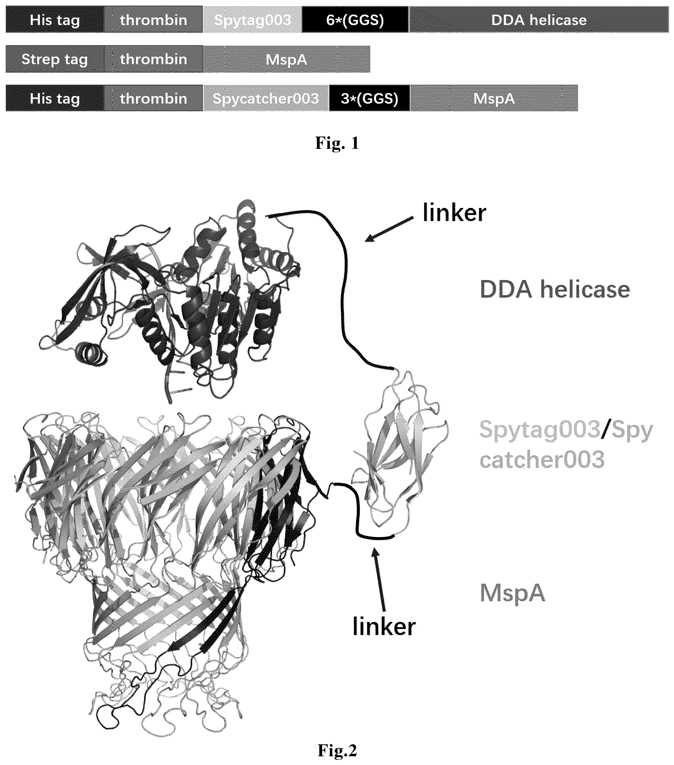

According to the schematic diagram of expression vector construction as shown in Fig. 1, gene fragments were designed and synthesized. 6*His-Spytag003-DDA and 6*His-Spycatcher003-MspA were cloned into the ampicillin-resistant pET.28a(+) vector to obtain recombinant plasmids pET.28a(+)-6*His-Spytag003-DDA and pET.28a(+)-6*His-Spycatcher003-MspA, respectively. Strep-MspA was cloned into the kanamycin-resistant pET.21a(+) vector to obtain the recombinant plasmid pET.21a(+)-strep-MspA.

### 2. Transformation of recombinant plasmid and large-scale expression of target protein

(1) The recombinant plasmid pET.28a(+)-6*His-Spytag003-DDA was transformed into DE3 competent cells, and a single colony was picked out, inoculated into 5 mL of LB medium containing kanamycin, and cultured under shaking at 37°C overnight. Then it was transferred to 1 L of LB medium, cultured under shaking at 37°C until OD₆₀₀=0.6 to 0.8, cooled to 16°C, and added with IPTG at a final concentration of 500 µM to induce expression overnight. The bacterial cells were collected.
(2) The recombinant plasmids pET.28a(+)-6*His-Spycatcher003-MspA and pET.21a(+)-strep-MspA were co-transformed into DE3 competent cells. A single colony was picked out, inoculated into 5 mL of LB medium containing two antibiotics, kanamycin and ampicillin, and cultured under shaking at 37°C overnight. Then, it was transferred to 1 L of LB medium, cultured under shaking at 37°C until OD₆₀₀=0.6 to 0.8, cooled to 16°C, and added with IPTG at a final concentration of 500 µM to induce expression overnight. The bacterial cells were collected.

### 3. Purification of Spytag003-DDA

The bacterial cells obtained in the above step 2 (1) were crushed under high pressure and purified through nickel column, ion exchange column, size exclusion chromatography and other methods, and finally the Spytag003-DDA protein with good purity was obtained.

### 4. Purification of complex of Spycatcher003-MspA and MspA in a ratio of 1:7

In the present application, an MspA octamer which only contains one molecule of Spycatcher003 was obtained through the following method. First, the bacterial cells co-expressing 6*His-Spycatcher003-MspA and Strep-MspA obtained in the above step 2 (2) were disrupted by high pressure (Lysis Buffer: 150 mM NaCl, 20 mM Tris, 1% DDM) and purified by Strep column (Wash Buffer: 150 mM NaCl, 20 mM Tris, 0.05% Tween 20; Elution buffer: 150 mM NaCl, 20 mM Tris, 10 mM desthiobiotin) to obtain complexes with different ratios of the two proteins. According to the different charges and the different affinities with nickel column, the complexes with different proportions of the two proteins were separated by the imidazole concentration gradient elution (imidazole concentration 25 mM to 250 mM) with the nickel column and the salt ion concentration gradient elution (BufferA: 100 mM; BufferB: 1000 mM NaCl) with the ion exchange column to obtain the MspA octamer formed by Spycatcher003-MspA and MspA in a ratio of 1:7, as shown in Fig. 3. From this, the Spycatcher003-labeled MspA octamer was obtained.

### 5. Covalent recombination of motor protein and porin complex

The Spycatcher003-labeled MspA octamer obtained in the above step 4 was mixed with the Spytag003-DDA obtained in the above step 3 at a ratio of 1:1.2, and reacted at 25°C for 30 minutes, the Superose 6 increase is used for size exclusion chromatography (Purification Buffer: 150 mM NaCl, 20mM Tris, 0.05% Tween20) to remove excess Spytag003-DDA protein, and the DDA-Spytag/Spycatcher-MspA complex was collected. The results were shown in Figs. 4 and 5.

### Example 2: Construction of nanopore biosensor and use thereof in sequencing

### (1) Construction of double-stranded polynucleotide sequencing library

### Materials and Methods:

Top strand of adapter (SEQ ID 10)
Bottom strand of adapter (SEQ ID 11)
Tether sequence (SEQ ID 12)
PUC57 vector of DNA sequence to be tested (SEQ ID 13)
First, the DNA to be tested (2µg dosage) was subjected to the End Repair: the reagents in Table 2 were mixed well, placed in a PCR machine at 20°C for 10min; 65°C for 10min, and then magnetic bead-based purification was carried out.

**Table 2: End Repair system**

| | |
|---|---|
| NEB Next FFPE DNA Repair Buffer | 3.5µL |
| NEB Next FFPE DNA Repair Mix | 2µL |
| Ultra II End-prep reaction Buffer | 3.5µL |
| Ultra II End-prep enzyme Mix | 3µL |
| DNA sample | 48µL |

The adapter is formed by annealing the adapter top strand (SEQ ID 10) and bottom strand (SEQ ID 11).

Then the product was ligated to the adapter according to the system as shown in Table 3 at 25°C for 30 minutes, and then magnetic bead-based purification was performed.

**Table 3: Ligation system**

| | |
|---|---|
| Adapter | 5µL |
| DNA sample (the above End Repair product + adapter) | 60µL |
| NEB Ligation Buffer | 25µL |
| NEB Next Quick T4 DNA Ligase | 10µL |

Finally, the unconnected adapters and DNA fragments were digested with enzymes according to the system as shown in Table 4, at 37°C for 5 minutes, and then magnetic bead-based purification was performed to complete the library construction.

**Table 4: Enzyme digestion system**

| | |
|---|---|
| NEB Buffer 4 | 2.5µL |
| T7 Exonuclease | 0.5µL |
| RNase Free H₂O | 2µL |
| DNA sample | 20µL |

### (1) Nanopore biosensor construction and sequencing

The motor protein Spytag003-DDA and the porin Spycatcher003-MspA(MspA)₇ were incubated in 1×PBS (Sangon, E607020-0500) at an equimolar ratio for 30 min (temperature: 25°C) to form a transmembrane protein-motor protein complex. Under the electric field force (applied voltage 0.18V), the transmembrane protein-motor protein complex was inserted into the prepared phospholipid bilayer membrane to form a single-channel nanopore. Patch clamp or other electrical signal collector was used to collect current signals (for methods, please refer to: Ji, Zhouxiang, and Peixuan Guo. "Channel from bacterial virus T7 DNA packaging motor for the differentiation of peptides composed of a mixture of acidic and basic amino acids." Biomaterials 214 (2019): 119222.). The open pore current from nanopores was also different under different voltages; and thus, it was possible to determine whether a single channel was formed according to the magnitude of the open pore current or the conductance of nanopores. Fig. 6 showed the open pore current of a single nanopore in a buffer (0.3 M KCl, 25 mM HEPES, 1 mM EDTA, 5 mM ATP, 25 mMgCl₂), wherein the open pore current at 50mV was about 35 pA; the open pore current at 100mV was about 75pA; the open pore current at 140mV was about 110pA; and the open pore current at 180mV was about 150pA.

After the single channel was formed, the double-stranded polynucleotide sequencing library constructed above was added into the chamber.

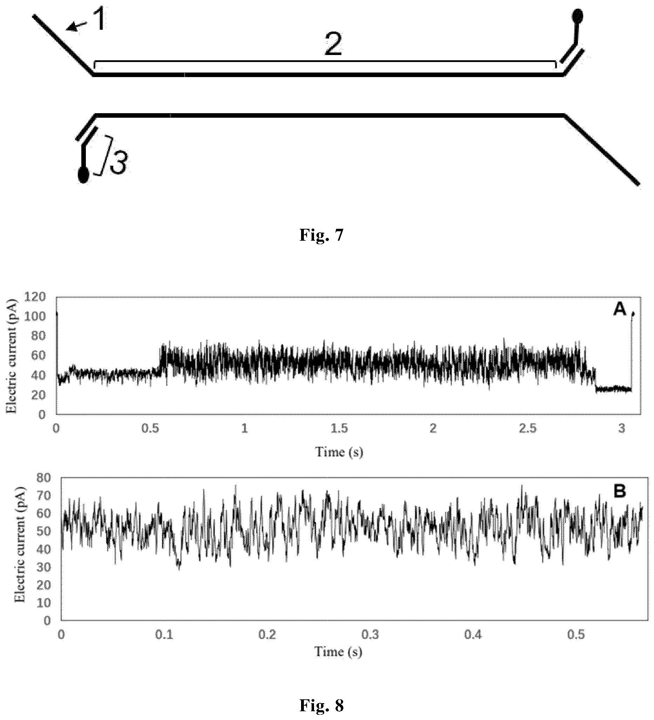

2 µg of the double-stranded polynucleotide sequencing library and 6 µL of the tether (concentration: 1 µM) were added to 300 µL of a sequencing buffer (0.3 M KCl, 25mM HEPES, 1mM EDTA, 5mM ATP, 25m MgCl₂), and added to the nanopore system. The schematic diagram of the double-stranded polynucleotide sequencing library molecule bound to the tether was shown in Fig. 7. Under the electric field force, the 5'-end overhang of the double-stranded polynucleotide sequencing library was captured by the transmembrane protein-motor protein complex and passed through the nanopore, so that the amplitude change of the current was observed. The complex could continuously capture the double-stranded polynucleotides and returned to the open pore current, the polynucleotides were identified as they moved and passed through the nanopore, and the current signals were shown in Fig. 8 (voltage: 0.14V). Panel A in Fig. 8 showed the current amplitude diagram of the sequencing event, and panel B in Fig. 8 showed a zoomed diagram of the sequencing event in panel A of Fig. 8. The results showed that the motor protein in the complex could unwind the two strands of the double-stranded polynucleotide and control the movement of the single-stranded polynucleotide through the pore formed by the complex, thereby completing the sequencing of polynucleotide.

Although the specific embodiments of the present application have been described in detail, those skilled in the art will understand that various modifications and changes can be made to the details based on all teachings that have been disclosed, and these changes are within the protection scope of the present application. The full scope of the present application is given by the appended claims and any equivalents thereof.

## Claims

1. A construct comprising a transmembrane protein pore subunit and a polynucleotide-binding protein, wherein the subunit retains its pore-forming ability, the polynucleotide-binding protein is capable of unwinding the two strands of a double-stranded polynucleotide and/or controlling the movement of a single-stranded polynucleotide through the pore; and, the subunit is in covalent binding to the polynucleotide-binding protein;
preferably, the covalent bonding is not via a peptide bond generated by nucleic acid translation;
preferably, the covalent binding is via an isopeptide bond;
preferably, the covalent binding occurs through chemical ligation (e.g., by click chemistry).

2. The construct according to claim 1, wherein the subunit and the polynucleotide-binding protein are bound via an isopeptide bond.

3. The construct according to claim 1 or 2, wherein the isopeptide bond is formed from a protein-protein binding pair, and the protein-protein binding pair consists of a first member (e.g., a first peptide tag) and a second member (e.g., a second peptide tag), wherein the first member and the second member are linked via an isopeptide bond, and, the first member is linked to the N-terminal or C-terminal of the transmembrane protein pore subunit optionally via a first linker (e.g., a rigid or flexible linker, such as a peptide linker comprising one or more glycines and/or one or more serines) to form a first component, and the second member is linked to the N-terminal or C-terminal of the polynucleotide-binding protein optionally via a second linker (e.g., a rigid or flexible linker, such as a peptide linker comprising one or more glycines and/or one or more serines) to form a second component;
preferably, the protein-protein binding pair is selected from the group consisting of: SpyCatcher/SpyTag pair, SpyTag002/SpyCatcher002 pair, SpyTag003/SpyCatcher003 pair, isopeptag-N/pilin-N pair, isopeptag/pilin-C pair, and SnoopTag/SnoopCatcher pair.

4. The construct according to any one of claims 1 to 3, wherein the isopeptide bond is formed by a SpyTag003/SpyCatcher003 pair;
preferably, the SpyCatcher003 is linked to the N-terminal of the subunit;
preferably, the SpyTag003 is linked to the N-terminal of the polynucleotide-binding protein;
preferably, the SpyCatcher003 has the amino acid sequence as set forth in SEQ ID NO: 4; preferably, the SpyTag003 has the amino acid sequence as set forth in SEQ ID NO: 1.

5. The construct according to any one of claims 1 to 4, wherein the subunit is selected from subunits derived from hemolysin, MspA, Frac, ClyA, PA63, CsgG, GspD, XcpQ, Wza, SP1, Phi29 connector, SPP1 connector, T3 connector, T4 connector, T7 connector, potassium ion channel protein, sodium ion channel protein, calcium ion channel protein;
preferably, the subunit is further linked with an additional polypeptide, and the additional polypeptide is selected from the group consisting of tag, enzyme cleavage site, signal peptide, guide peptide, detectable label, and any combination thereof;
preferably, the subunit has the amino acid sequence as set forth in SEQ ID NO: 3 or 17.

6. The construct according to any one of claims 1 to 5, wherein the polynucleotide-binding protein is selected from the group consisting of nucleic acid helicases, such as DNA helicases or RNA helicases;
preferably, the polynucleotide-binding protein is selected from Dda, UvrD, Rep, RecQ, PcrA, eIF4A, NS3, Rep, gp41 and T7gp4;
preferably, the polynucleotide-binding protein is further linked with an additional polypeptide, and the additional polypeptide is selected from the group consisting of tag, enzyme cleavage site, signal peptide, guide peptide, detectable label, and any combination thereof;
preferably, the polynucleotide-binding protein has the amino acid sequence as set forth in SEQ ID NO: 2 or 16.

7. A pore for sequencing a polynucleotide, which comprises at least one the construct according to any one of claims 1 to 6;
preferably, the pore is a transmembrane protein pore.

8. The pore according to claim 7, which comprises at least one the construct according to any one of claims 1 to 6 and other subunit required to form the pore;
preferably, the pore comprises a sufficient number of other subunits required to form the pore;
preferably, the other subunit required to form the pore is the same as or different from the transmembrane protein pore subunit in the construct;
preferably, the other subunit required to form the pore is the same as the transmembrane protein pore subunit in the construct, or the other subunit required to form the pore is a paralog, homologue or variants of the transmembrane protein pore subunit in the construct.

9. The pore according to claim 7 or 8, which comprises the construct according to any one of claims 1 to 6 and other subunit required to form the pore;
preferably, the pore comprises a sufficient number of other subunits required to form the pore;
preferably, the pore is formed from one construct and seven other subunits required to form the pore;
preferably, the other subunit required to form the pore is the same as or different from the transmembrane protein pore subunit in the construct;
preferably, the other subunit required to form the pore is the same as the transmembrane protein pore subunit in the construct, or the other subunit required to form the pore is a paralog, homologue or variants of the transmembrane protein pore subunit in the construct.

10. An isolated nucleic acid, which encodes the construct according to any one of claims 1 to 6.

11. The isolated nucleic acid molecule according to claim 10, wherein the isolated nucleic acid molecule comprises a first nucleotide sequence encoding a first component and a second nucleotide sequence encoding a second component, the first component and the second component are as defined in claim 3 or 4, and the first nucleotide sequence and the second nucleotide sequence are present on the same or different isolated nucleic acid molecules;
preferably, the first component is a first protein formed by linking the first member to the N-terminal or C-terminal of the transmembrane protein pore subunit optionally via a first peptide linker; and the second component is a second protein formed by linking the second member to the N-terminal or C-terminal of the polynucleotide-binding protein via a second peptide linker.

12. A vector, which comprises the isolated nucleic acid molecule according to claim 10 or 11; preferably, the vector is a cloning vector or an expression vector.

13. A host cell, which comprises the isolated nucleic acid molecule according to claim 10 or 11 or the vector according to claim 12.

14. A method for preparing the pore according to any one of claims 7 to 9, the method comprising the following steps:
(1) contacting a first component with other subunit required to form the pore to form a multimer, the multimer being a pore that does not contain a polynucleotide-binding protein, wherein the first component is as defined in claim 3 or 4, the other subunit required to form the pore is as defined in claim 8 or 9;
(2) contacting the multimer with a second component, the second component being as defined in claim 3 or 4, so that the second component is linked to the first component in the multimer via an isopeptide, to form the pore linked with a polynucleotide-binding protein.
preferably, the multimer in step (1) is an octamer formed from one first component and seven the other subunits required to form the pore;
preferably, the subunit in the first component is the same as the other subunit required to form the pore;
preferably, step (1) comprises the following steps: culturing a host cell containing a first nucleotide sequence encoding the first component and a third nucleotide sequence encoding the other subunit required to form the pore to obtain the pore that does not contain a polynucleotide-binding protein;
preferably, step (1) further comprises a step of purifying the pore that does not contain a polynucleotide-binding protein.

15. A method for preparing the pore according to any one of claims 7 to 9, the method comprising the following steps:
(1) providing the construct according to any one of claims 1 to 6;
(2) incubating the construct with other subunit required to form the pore to form a multimer, in which the multimer is the pore linked with a polynucleotide-binding protein;
preferably, in step (2), one or more of the constructs are incubated with one or more other subunits required to form the pore;
preferably, the multimer in step (1) is an octamer formed from one construct and seven the other subunits required to form the pore.

16. A method for sequencing a target polynucleotide, comprising:
(a) providing the pore according to any one of claims 7 to 9, and a polynucleotide to be tested;
(b) contacting the polynucleotide to be tested with the pore, so that the polynucleotide-binding protein of the pore binds to the polynucleotide to be tested, and the polynucleotide-binding protein controls a single-stranded polynucleotide to which it binds to pass through the pore; and,
(c) obtaining one or more measurement values as the single-stranded polynucleotide moves relative to the pore, wherein the one or more measurement values are capable of being used to character sequence information of the polynucleotide to be tested.

17. The method according to claim 16, wherein, in step (a), the polynucleotide to be tested is a double-stranded polynucleotide, and the polynucleotide-binding protein unwinds the double-stranded polynucleotide to provide a single-stranded polynucleotide, and the polynucleotide-binding protein controls the single-stranded polynucleotide to pass through the pore; the double-stranded polynucleotide comprises at least one single-stranded overhang (e.g., a 5'-end overhang and/or a 3'-end overhang), and the single-stranded overhang comprises a leader sequence, and the leader sequence guides the single-stranded polynucleotide to which it links to enter the pore.

18. The method according to claim 17, wherein the polynucleotide-binding protein controls the single-stranded polynucleotide to move through the pore in the direction from 5' end to 3' end, and the double-stranded polynucleotide comprises at least one 5'-end overhang, and the 5'-end overhang comprises the leader sequence.

19. The method according to claim 17, wherein the polynucleotide-binding protein controls the single-stranded polynucleotide to move through the pore in the direction from 3' end to 5' end, and the double-stranded polynucleotide comprises at least one 3'-end overhang, and the 3'-end overhang comprises the leader sequence.

20. The method according to any one of claims 17 to 19, wherein the polynucleotide to be tested is a linear double-stranded polynucleotide;
preferably, the linear double-stranded polynucleotide comprises at least one single-stranded overhang (e.g., a 5'-end overhang and/or a 3'-end overhang) at each of its two ends, and the single-stranded overhang comprises the leader sequence;
preferably, the linear double-stranded polynucleotide comprises at least one single-stranded overhang (e.g., a 5'-end overhang and/or a 3'-end overhang) at its one end, and the single-stranded overhang comprises the leader sequence; and, the linear double-stranded polynucleotide comprises a bridge portion at its other end, and the bridge portion covalently links the two single strands of the linear double-stranded polynucleotide.

21. The method according to any one of claims 17 to 19, wherein the polynucleotide to be tested is a circular double-stranded polynucleotide;
preferably, the circular double-stranded polynucleotide is obtained by rolling circle amplification using a primer containing the leader sequence and a carrier sequence as a template.

22. The method according to claim 16, wherein, in step (a), the polynucleotide to be tested is a single-stranded polynucleotide, and the polynucleotide-binding protein controls the single-stranded polynucleotide to move through the pore; wherein, the single-stranded polynucleotide comprises a leader sequence at least at one of its two ends, and the leader sequence guides the single-stranded polynucleotide to which it links to enter the pore.

23. The method according to claim 22, wherein the polynucleotide-binding protein controls the single-stranded polynucleotide to move through the pore in the direction from 5' end to 3' end, and the single-stranded polynucleotide comprises the leader sequence at least at its 5' end.

24. The method according to claim 22, wherein the polynucleotide-binding protein controls the single-stranded polynucleotide to move through the pore in the direction from 3' end to 5' end, and the single-stranded polynucleotide comprises the leader sequence at least at its 3' end.

25. The method according to any one of claims 16 to 24, wherein the polynucleotide to be tested is linked to the outer edge or vicinity of the pore;
preferably, one or more tethers are conjugated to the outer edge or vicinity of the pore, and the tether comprises a capture sequence having sequence complementarity with a portion of the polynucleotide to be tested, and the capture sequence is capable of hybridizing with a complementary portion in the polynucleotide to be tested so that the polynucleotide to be tested can be linked to the outer edge or vicinity of the pore;
preferably, when the polynucleotide to be tested is as defined in any one of claims 17 to 21, the complementary portion in the polynucleotide to be tested comprises a portion of the single-stranded overhang or a portion of a strand that is aligned with the single-stranded overhang;
preferably, when the polynucleotide to be tested is as defined in any one of claims 22 to 24, the complementary portion in the polynucleotide to be tested comprises a portion of the single-stranded polynucleotide.

26. The method according to any one of claims 16 to 25, wherein the pore is disposed in a membrane;
preferably, the membrane is an amphiphilic layer, such as a lipid bilayer (e.g., phospholipid bilayer) or a high molecular polymer membrane (e.g., di-block polymer membrane, tri-block polymer membrane).

27. The method according to any one of claims 16 to 26, wherein step (b) and step (c) are performed in a solution;
preferably, the solution is ionic and the measurement value is an ionic current through the nanopore.

28. The method according to any one of claims 16 to 27, wherein in step (b), a potential difference is provided across the nanopore to allow the single-stranded nucleic acid to enter the nanopore.

29. A device for sequencing a target polynucleotide, comprising: (i) a membrane; (ii) a plurality of transmembrane protein pores in the membrane, the transmembrane protein pores being selected from the pore according to any one of claims 7 to 9;
preferably, the device further comprises instructions for carrying out the method according to any one of claims 16 to 28;
preferably, the membrane is an amphiphilic layer, such as a lipid bilayer (e.g., phospholipid bilayer) or a high molecular polymer membrane (e.g., di-block polymer membrane, tri-block polymer membrane);
preferably, the device does not comprise a polynucleotide-binding protein provided separately from (ii).

30. A kit, which comprises the construct according to any one of claims 1 to 6, the pore according to any one of claims 7 to 9, the isolated nucleic acid molecule according to claim 10 or 11, the vector according to claim 12, and/or, the host cell according to claim 13;
preferably, the kit further comprises a reagent for nanopore sequencing.

31. Use of the construct according to any one of claims 1 to 6, the pore according to any one of claims 7 to 9, the isolated nucleic acid molecule according to claim 10 or 11, the vector according to claim 12, the host cell according to claim 13, the device according to claim 29, or the kit according to claim 30 for sequencing a polynucleotide.
